# EUROPEAN PATENT APPLICATION

(11) **EP 2 141 249 A1**
(43) Date of publication of application: **06.01.2010**
(21) Application number: 08305362.9
(22) Date of filing: 30.06.2008
(51) Int. Cl.: C12Q 1/68

(54) **Method for the detection of specific nucleic acid hybridization**

(71) Applicant: Institut National De La Sante Et De La Recherche Medicale, 75013 Paris (FR)
(72) Inventor: Curmi, Patrick, 91025 Evry Cedex (FR); Pastre, David, 91025 Evry Cedex (FR); Hamon, Loïc, 91025 Evry Cedex (FR)
(74) Representative: Jacobson, Claude

(57) **Abstract**

The present invention relates to the use of a negatively charged surface, on which at least one single stranded nucleic acid probe is attached at several points along its length, to specifically hybridize the probe with at least one nucleic acid target.

## Description

### Field of the invention

The present invention relates to methods for detecting nucleic acids by specific hybridization.

### Background of the invention

DNA- and RNA-based microarray and biosensor technologies are widely used for genotyping, studying gene expression, or detecting biological compounds (see *e.g.* Sassolas et al. (2008) Chem. Rev. 108:109-139; Copland et al. (2003) Rec. Prog. Hormone Res. 58:25-53; Cloarec et al. (2008) IRBM-RBM. 29(2-3):105-127; Dufva (2005) Biom. Eng. 22:173-184).

Typically, current microarray-based technologies are underpinned by solid-phase hybridization or "surface" hybridization, in which nucleic acid strands tethered on a solid support, referred to as "probes", associate to single stranded DNA (ssDNA) or single stranded RNA (ssRNA) molecules from a solution, referred to as "targets".

Usually the probes are immobilized on microscopic spots via ionic, covalent or avidin-biotin attachments and arrayed by contact or non-contact printing on a solid support. Alternatively, oligonucleotide probes can be directly synthesized on the solid support by using lithographic masks. Nucleic acid hybridization can be detected according to various techniques, notably based on optical, electrochemical or gravimetric methods. Several factors, such as ionic strength, sequence composition of the probes and targets, or temperature, which affect surface hybridization, have been extensively studied using techniques which notably include optical, spectroscopic, electrochemical and mechanical methods.

However, even though the field of microarray technology has reached maturity, detection and analysis of low levels of targets, such as mRNA, remains a difficult issue, which notably impairs the development of microarray-based diagnostic methods. Thus, gene expression quantification generally includes Northern blotting or ribonuclease protection assays (RPA) which techniques lack adequate sensitivity. Besides, where reverse transcription-polymerase chain reaction (RT-PCR) is used, a better sensitivity can be achieved, but the optimization of the PCR-primers is time-consuming. Furthermore, the relative proportion of nucleic acids after amplification may not reflect the population existing in the starting mixture, in particular due to selective and non linear target amplification.

Yet another limitation of the current microarrays lies in the long hybridization step owing to the sterical hinderance on the solid support due to the high density of probes. Consequently, hybridization with microarrays can take from 4 hours to 24 hours, which considerably slows down the methods using microarrays.

Accordingly, there is a need for methods for detecting specific hybridization of nucleic acids, in particular at low concentrations of targets, which are more rapid and/or sensitive than current methods. This would notably open the way to the development of microarray-based diagnostic methods.

Atomic force microscopy (AFM) has been used for imaging, in particular at the single molecule level, double stranded DNA (dsDNA) and single stranded DNA-protein complexes on ultraflat substrates (Allison et al. (1996) Proc. Natl. Acad. Sci. USA 93:8826-8829; van Noort et al. (1998) Biophys. J. 74:2840-2849). This microscope generates a three-dimensional image by probing the sample surface with a sharp tip attached to the end of a flexible cantilever. As AFM is a surface technique, it is necessary that nucleic acid molecules are attached to the surface so that they can be imaged reproducibly and with high resolution.

The most popular substrate in this respect is muscovite mica, a highly negatively charged surface. However, the spreading of nucleic acids on an atomically flat surface is not an easy task. Accordingly, specific methods have been developed which enable adsorbing both single-stranded and double-stranded nucleic acid molecules on mica, in particular by using spermidine, a trivalent polyamine which allows adsorption of nucleic acids on mica at both low and high ionic strengths (Hamon et al. (2007) Nucleic Ac. Res. 35:e58).

### Summary of the invention

The present invention arises from the unexpected finding by the inventors, that adsorption of ssDNA on a mica surface allows for hybridization of complementary strands, in spite of the multiple attachment points which link the ssDNA to the surface and constitute an obstacle to ssDNA movements required for hybridization. This notably enables the detection of hybridization of low amounts of nucleic acids, in particular using Atomic Force Microscopy. In addition, the inventors have shown that rapid probe and target diffusion could occur on the surface, which enabled to significantly reduce incubation time necessary for hybridization. Methods making use of such surfaces would be particularly interesting in the diagnostic field.

Thus, the present invention relates to the use of a negatively charged surface, on which at least one single stranded nucleic acid probe is attached at several points along its length, to specifically hybridize the probe with at least one nucleic acid target.

The present invention also relates to a method for specifically hybridizing at least one single stranded nucleic acid probe with at least one nucleic acid target, wherein the probe is attached at several points along its length on a negatively charged surface.

The present invention also relates to a method for detecting a nucleic acid target in a composition, comprising:
- contacting a composition liable to contain the nucleic acid target, with a surface as defined above,
- washing the surface so as to discard nucleic acid targets which are not attached to the surface,
- allowing hybridization of the nucleic acid target with a single-stranded nucleic acid probe to proceed,
- determining if hybridization of the nucleic acid target with a single-stranded nucleic acid probe has occurred, whereby, if hybridization has occurred, the nucleic acid target is detected.

### Detailed description of the invention

### Negatively charged surface

As used herein, a "negatively charged surface" refers to any plane surface displaying a net negative charge and liable to bind, directly or indirectly, to biological molecules such as nucleic acids. The negatively charged surface can either be of synthetic or natural origin, or be made of an organic or inorganic material.

However, it is preferred that the negatively charged surface is a mineral, in particular an aluminosilicate, such as mica. Mica is of particular interest for use according to the present invention because, thanks to its perfect cleavage planes, it represents an almost ideal surface for atomic force microscopy. Particular micas notably encompass muscovite, biotite, phlogopite, and lepidolite.

### Nucleic acid probe

In the context of the invention, a "probe" refers to a nucleic acid able to bind in a base-specific manner to a complementary strand of nucleic acid.

"Nucleic acid" refers herein to both RNA and DNA, including cDNA, genomic DNA, and synthetic DNA. Nucleic acids can have any three-dimensional structure. A nucleic acid can be double-stranded or single-stranded. Non-limiting examples of nucleic acids include genes, gene fragments, exons, introns, messenger RNA (mRNA), transfer RNA (tRNA), ribosomal RNA (rRNA), siRNA, micro-RNA, ribozymes, cDNA, recombinant nucleic acids, and branched nucleic acids. A nucleic acid may contain unconventional or modified nucleotides.

As used herein, the single-stranded nucleic acid probe of the invention may be labelled with a detectable moiety, *i*.*e*. a moiety capable of generating a detectable signal, such as a radioactive, calorimetric, fluorescent, chemiluminescent or electrochemiluminescent signal. Numerous such detectable moieties are known in the art. By way of example, the moiety may be a radioactive compound or a detectable enzyme (*e.g*., horseradish peroxidase (HRP)).

Preferably, the single-stranded nucleic acid probe according to the invention comprises or is constituted of from about 5 to about 2000 nucleotides. More preferably, the single-stranded nucleic acid probe comprises or is constituted of from 100 to 2000 nucleotides.

A plurality of single-stranded nucleic acid probes according to the invention may be attached to the negatively charged surface according to the invention. Where this is the case, the single-stranded nucleic acid probes are preferably separated from one another by a minimal step of 60 bp.

Besides, the inventors have demonstrated that the use of AFM to observe DNA molecules adsorbed on mica surface enabled simultaneous detection of several different probes, which could lead to an ultra-miniaturisation of devices using this technique.

Accordingly, it is preferred that at least two different single-stranded nucleic acid probes are attached to the negatively charged surface. More preferably, from 2 to 10 different single-stranded nucleic acid probes are attached to the negatively charged surface.

Most preferably, a plurality of different single-stranded nucleic acid probes according to the invention, where each individual single-stranded nucleic acid probe is present in several copies, is attached the negatively charged surface according to the invention.ln particular, it is preferred that the several copies of an individual single-stranded nucleic acid probe are attached on a same spot of the negatively charged surface. The amount of single-stranded nucleic acid probes deposited on a spot is preferably of about 10 pg for a spot with a radius of 100 µm, or of about 10⁷ single-stranded nucleic acid probes/cm².

### Attachment

As intended herein, the expressions "attached at several points along its length" and "attached" are used indifferently and indicate that essentially the whole length of the single-stranded nucleic acid probe according to the invention is adsorbed on the negatively charged surface according to the invention.

Preferably, attachment of the probe to the surface is mediated by non-covalently bound cations. As used herein, the expression "non-covalently bound cations" indicates that the cations are bound by non-covalent bonds to the single-stranded nucleic acid probe and to the negatively charged surface. It is preferred that the non-covalent bonds are weak bonds, such as electrostatic, hydrophobic or Van der Waals bonds. Weak bonds are advantageous in that they allow probe diffusion on the surface and enhance probe accessibility to target.

As intended herein, the term "cations" refers to positively charged atoms or molecules. Cations according to the invention may be in particular selected in the group consisting of monovalent, divalent, trivalent or tetravalent cations.

Examples of monovalent cations according to the invention include Li⁺, Na⁺, or K⁺. Examples of divalent cations according to the invention include Mg²⁺, Ca²⁺, Cu²⁺, Zn²⁺, Mn²⁺; Ni²⁺, and putrescine. Examples of trivalent cations according to the invention include Al³⁺, Fe³⁺, spermidine (Spd³⁺) or cobalt hexamine (CoHex³⁺). Spermine (Spm⁴⁺) is an example of a tetravalent cation according to the invention.

More preferably, cations according to the invention are selected from the group consisting of Na⁺, K⁺, Li⁺, Zn²⁺, putrescine, Al³⁺, Fe³⁺, Cobalt hexamine, Mg²⁺, Mn²⁺, Ni²⁺, Ca²⁺, Cd ²⁺ spermidine, and spermine. Spermidine is of particular interest because it can induce a strong DNA adsorption even at submillimolar concentration and can still be effective at high NaCl concentration (Hamon et al. (2007) Nucleic. Acids. Res. 35:e58).

By way of example, in order to be attached to the negatively charged surface, the single-stranded nucleic acid probes of the invention can be incubated for about 1 minute with the surface according to the invention, for instance in the presence of a multivalent cation as defined above, in particular a trivalent cation such as spermidine. Preferably, the adsorption is carried out in a solution containing 4 µM spermidine at 95°C. The surface on which nucleic acid probes are attached may then be washed in a fresh solution of the same composition.

### Nucleic acid target

As used herein, a "nucleic acid target" refers to any naturally occurring or synthetic polymer of nucleotides, such as single or double stranded DNA, RNA. In particular the nucleic acid target may originate or derive from rRNA, mRNA, plasmidic DNA, bacterial DNA, viral DNA, viral RNA, and chromosomal DNA.

Preferably, the nucleic acid target according to the invention is single-stranded.

Advantageously and contrary to current technologies using fluorescence to detect hybridization, detection of a single molecule of isolated target-probe complexes is possible according to the invention. Very small amounts of target material to be detected are therefore needed. For example, about 10 pg of nucleic acid targets could be used, which would represent 10⁷ target molecules/cm².

### Hybridization

The terms "hybridize" or "hybridization" refer herein to the binding of a single-stranded nucleic acid molecule to another single-stranded nucleic acid molecule which binding involves the formation of base pairs.

It is preferred that the quantity of nucleic acid targets liable to be contained in the above-defined composition is equal to the sum of the nucleic acid probes attached on the surface.

The above-defined contacting step may be carried in any conditions suitable for nucleic acid attachment to the above-defined negatively charged surface. Such conditions may be easily defined by the man skilled in the art. In particular, the above-defined contacting step may be performed in a solution comprising a divalent, trivalent or tetravalent cation, such as spermidine, preferably at a concentration from about 1 µM to about 100 µM, more preferably from about 2 µM to about 10 µM, and most preferably of about 4 µM. It is preferably performed at a temperature above 50°C, more preferably at a temperature above 65°C, and most preferably at a temperature of about 95°C. Preferably also, the contacting step is carried for less than 5 minutes, more preferably for less than 2 minutes, and most preferably for about 1 minute. Most preferably the contacting step is thus conducted for 1 minute in a solution containing 4 µM spermidine, at a temperature of 95°C.

The above-defined washing step is to be carried in conditions suitable for removing nucleic acid targets which are not attached to the above-defined negatively charged surface. Such conditions can be easily defined by the man skilled in the art. In particular, the conditions used for the washing step can be identical to the conditions used for the contacting step. Thus, the above-defined washing step may be performed in a solution comprising a divalent, trivalent or tetravalent cation, such as spermidine, preferably at a concentration from about 1 µM to about 100 µM, more preferably from about 2 µM to about 10 µM, and most preferably of about 4 µM. It is preferably performed at a temperature above 50°C, more preferably at a temperature above 65°C, and most preferably at a temperature of about 95°C. Preferably also, the washing step is carried for less than 5 minutes, more preferably for less than 2 minutes, and most preferably for about 1 minute. Most preferably the washing step is thus conducted for 1 minute in a solution containing 4 µM spermidine, at a temperature of 95°C.

The above-defined hybridization step can be carried in any conditions allowing nucleic acid hybridization to proceed. Such conditions can be easily defined by the man skilled in the art. In particular, the above-defined hybridization step may be carried out in a solution comprising monovalent and/or divalent and/or trivalent cations, such as a solution comprising from about 1 mM to about 100 mM MgCl₂ and from about 0 mM to 200 mM NaCl. It is performed at a temperature preferably above 50°C, and preferably below 95 °C. More preferably, it is performed at a temperature of about 65°C. Preferably, the hybridization step is carried for less than 30 minutes, more preferably for less than 15 minutes, even more preferably for less than 10 minutes. Preferably, the hybridization step is carried for at least 5 minutes. The hybridization step is carried at a pH preferably above 7 and preferably below 8. In particular, the above-defined hybridization step can thus be carried out in a solution containing NaCl 50 mM and MgCl₂ 10 mM at 65°C and with Tris-HCl 20 mM, pH 7.5 for 5 minutes.

Advantageously, the hybridization process carried with the negatively charged surface according to the invention is greatly accelerated in comparison with technologies of the state of the art which usually require several hours of incubation. Without being bound to any particular theory, it is thought that the acceleration of hybridization is notably due to surface diffusion of nucleic acids on the negatively charged surface which increases the probability of encounter between a probe and its target.

Preferably, determining if hybridization has occurred is performed according to the invention by measuring the length of nucleic acid target-nucleic acid probe complexes with probes of different sizes which enables to distinguish them. Preferably, this step is performed using atomic force microscopy (AFM), fluorescence microscopy (FRET), Stimulated Emission Depletion microscopy (STED) or electronic microscopy. Most preferably, determining if hybridization of the nucleic acid target with a single-stranded nucleic acid probe has occurred is carried by AFM.

AFM should enable to detect differences of length of 20 nm (60 bp) between two probes. Analysis of about 50 target-probe complexes per spot and per probe according to the invention should therefore be sufficient to validate hybridization and the sequence of the nucleic acid target.

The step of determining if hybridization has occurred according to the invention may optionally be preceded by a drying step using nitrogen flow, filter paper or uranyl acetate. Most preferably, the drying step is carried by using a 0.02% uranyl acetate solution followed by drying with paper filter.

The invention will be further illustrated by the following figures and examples.

### Brief description of the figures

**Figure 1** shows the surface density of hybridized 1188 bp DNA on mica after 5 minutes incubation in 20 mM Tris-HCl, 50 mM NaCl, 10 mM MgCl₂ according to the hybridization temperature. The maximum is situated around 65°C.
**Figure 2** shows the surface density of hybridized 1188 bp DNA on mica according to the incubation time in magnesium buffer (black square) or in spermidine buffer (black circle).
**Figure 3** shows the surface densities of 231 bp (78 ± 10 nm), 377 bp (128 ± 10 nm), 639 bp (217 ± 10 nm), 1050 bp (357 ± 10 nm) and 2064 bp (702 ± 10 nm) double stranded DNAs deposited simultaneously on mica surface without denaturation process. The peak width corresponds with the AFM tip resolution associated with the measure of the DNA contour length by the software.
**Figure 4** shows the surface densities of 231 bp (78 ± 10 nm), 377 bp (128 ± 10 nm), 639 bp (217 ± 10 nm), 1050 bp (357 ± 10 nm) and 2064 bp (702 ± 10 nm) single stranded DNAs deposited simultaneously on mica surface with their complementary strands and hybridized on mica. The peak width after hybridization process is quite identical to those obtained without denaturation process for DNA fragments shorter than around 400 nm (*i.e.* 1150 bp).

### Example

This example describes the determination of the optimal conditions for hybridization between a nucleic acid target and a nucleic acid probe attached to a negatively charged surface to occur. It further demonstrates the effects of the DNA length on the efficiency of hybridization on mica and provides evidence of the possibility of using several probes on the same spot. In addition this example shows that the incubation time required for hybridization can be significantly reduced.

### Experimental procedure:

### Materials

The chemical compounds (MgCl₂, spermidine) and pBR322 plasmid were purchased from Sigma-Aldrich. The restriction enzymes were purchased from Sigma-Aldrich *(Pvu*II*, Eco*RV) and New England Biolabs (*Eco*RI*, Bam*HI, *Nde*I and *Bso*BI).

The 1188 bp fragments were obtained from pBR322 plasmid by PCR amplification with the forward primer 5'-CATAGCAGAACTTTAAAAGTACTCATC-3' (SEQ ID NO:1) and the reverse primer 5'-AGTTGCATGATAAAGAAGACAGTC-3' (SEQ ID NO:2). The PCR product was purified by PCR purification kit microcolumn (Qiagen, protocol as described by the manufacturer). The DNA was ethanol precipitated, dried and re-suspended in TE buffer (10 mM Tris-HCl pH 7.5, 1 mM EDTA)

The 639 bp and 3722 bp DNA fragments were produced by digesting plasmid pBR322 with restriction enzymes *Pvu*II and *Bso*BI in NEBuffer III. The 231, 377, 639, 1050 and 2064 bp DNA fragments were produced by digesting plasmid pBR322 with restriction enzymes *Eco*RI*, Bam*HI*, Bso*BI, *Pvu*II and *Nde*I in NEBuffer II. For longer fragments, pBR322 plasmids were linearized with restriction enzyme *Eco*RV.

### Detection of DNA hybridization on mica

A linearized plasmid DNA was first denaturated at high temperature for 4 min, thus leading to a solution containing both complementary strands at equimolar concentration in presence of spermidine cations. A freshly cleaved mica surface (0.5×1.5 cm²) was heated at the same temperature and plunged into the DNA solution during 40 seconds to adsorb the complementary fragments of ssDNA on mica. After a rinsing step with the incubation buffer at high temperature to remove unadsorbed ssDNA fragments, the mica surface was directly plunged into a 500 µL solution in which the hybridization between the two complementary fragments adsorbed on the mica surface could take place. This buffer solution contained 20 mM Tris-HCl, pH 7.5, 50 mM NaCl and different concentrations of divalent (MgCl₂) or trivalent (spermidine) cations. To investigate the effect of the temperature of incubation, it was varied in the range of 40 to 90°C. A low surface density of adsorbed ssDNA (about 4 ssDNA/µm²) was chosen to allow the detection of single hybridization events. The separation distance between two nearest complementary ssDNA molecules should thus be larger than the radius of gyration of the hybridized DNA to avoid their overlapping (distance larger than 200 nm for a 4000 bp plasmid).

After various incubation times, the mica surface was then rinsed with 0,02% diluted uranyl acetate solution in order to stabilize the DNA molecules in their 3D conformations for AFM imaging in air (Revet and Fourcade, (1998) Nucleic Acids Res. 26:2092-2097). The sample was then rapidly rinsed with pure water (Millipore) to obtain a clean surface after drying with filter paper and imaged by AFM.

### Atomic Force Microscopy

The sample imaging was performed in Tapping ModeTM with a MultimodeTM AFM (Veeco, Santa Barbara, CA) operating with a Nanoscope IIIaTM controller. Olympus (Hamburg, Germany) silicon cantilevers AC160TS with resonant frequencies of about 300 kHz and nominal spring constants between 10 to 100 N/m were used. The scan frequency was typically 1.5 Hz per line and the modulation amplitude was of about a few nanometers. Data were acquired at a set point chosen to minimize tip-sample interaction. Image analysis was performed on Nanoscope IIIaTM software. Only a first or second order polynomial function was used to remove the background slope.

### Control experiment

This control experiment was performed to observe whether DNA molecules were released or not from the surface into the solution during the incubation period.

After the removal of the mica surface on which DNA molecules were adsorbed and potentially hybridized, a control mica surface was plunged into the incubation buffer. Spermidine (100 µM) was then added to enhance DNA adsorption. After 1 min incubation, the inventors observed by AFM whether or not there was DNA molecules, previously desorbed from the mica surface, reabsorbed on the control mica. As no DNA molecule adsorbed on the control surfaces was observed, it indicated that desorption did not occur during the hybridization experiments on mica under the conditions reported herein. In addition, the inventors observed that the surface density of hybridized DNA remained constant during incubation time whatever the incubation temperature.

### Identification criterion of the hybridization efficiency

The efficiency of hybridization was represented by the surface density of completely hybridized DNA *via* the measurement of the length of the hybridized fragments by AFM. As the DNA length was measured with an accuracy of ± 20 nm using an image analysis software, the inventors considered a probe/target as fully hybridized when its contour length was equal to its theoretical length (number of base pair×0.34 ± 20 nm). For each data point, three different surfaces were analyzed and at least one hundred DNA molecules were measured for each surface. The mean value of the surface density of hybridized probes obtained for the three surfaces was reported. The detection of hybridization was performed on only 30 -100 hybridized probe/target, which was sufficient for a reliable detection.

The formation of multimolecular aggregates of partially hybridized DNA due to cross-hybridization was discarded for this analysis.

### Experimental results:

### Denaturation and adsorption of complementary strand at high temperatures

Before the study of the surface hybridization process, dsDNA molecules were denaturized and adsorbed on mica in their single-stranded state.

DNA molecules were diluted to a concentration of 0.3 µg/mL in a 300 µL solution containing 4 µM spermidine and heated at temperatures ranging from 88 to 92°C during 4 min. DNA molecules were incubated in solution at very low ionic strength (4 µM spermidine in pure water) to favour denaturation because there was a long ranged electric repulsion between negatively charged complementary strands (Tabor (1962) Biochemistry 1:496-501). The freshly cleaved mica surface (0.5×1.5 cm²) was heated to the same temperature, plunged into the DNA solution at the same temperature during 40 seconds to adsorb DNA on mica *i*.*e*. mediated by spermidine cations.

Below 88°C, adsorbed DNA molecules appeared to be double stranded although local openings of the double helix referred to as denaturation bubbles may also occur transiently at such temperatures. Above 88°C, the denaturation process could be clearly observed. Long DNA sequences appeared in their single stranded state. By increasing the ionic strength of the buffer (100 µM spermidine, NaCl 50 mM), the denaturation process occured at higher temperature (92°C) due to weaker electrostatic repulsion between complementary strands which induced an increase in the melting temperature. In addition, partial denaturation could be hardly distinguished at the dsDNA extremities because the self-repulsion of ssDNA strands was inhibited leading to low persistence length and thus to a globular shape.

In light of these results, DNA hybridization assays on a mica surface were performed after heating diluted DNA solutions at 95°C (even if 92°C seemed to be sufficient) in 4 µM spermidine ensuring that all molecules adsorbed on mica surface were isolated and in their single stranded state.

### Hybridization assays on mica surface at various temperatures

After the first step of denaturation and the deposition of the denaturized DNA molecules on the mica surface, a rinsing step at 95°C in the denaturing buffer (4 µM spermidine) was performed to remove unadsorbed fragments. The mica was then transferred into a reaction buffer (10 mM MgCl₂, 20 mM Tris-HCl pH 7.5, 50 mM NaCl) at various temperatures and incubated for 5 min to allow both DNA diffusion on the surface and hybridization of the complementary strands.

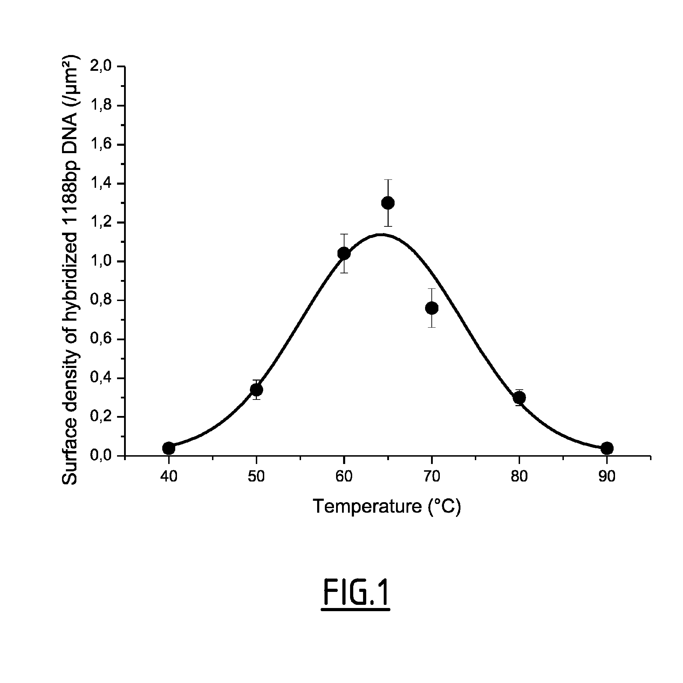

The results showed that DNA hybridization on the mica surface exhibited a typical pattern with an optimal temperature of about 65°C (**Figure 1**). Indeed, for the 1188 bp DNA fragment, the surface density of hybridized fragment was maximum at 65°C. Higher temperatures lowered the association probability as the two complementary strands were placed under stringent conditions whereas lower temperatures increased the probability of cross-hybridization process and thus reduced the surface density of fully hybridized fragments. Consequently, the next experiments were conducted at 65°C as the temperature classically used for DNA microarray experiments.

### Incubation time and counterion effects on DNA strand hybridization

The inventors chose to test the ability of two multivalent cations (magnesium and spermidine) to allow hybridization of ssDNA adsorbed on mica, owing to their ability to induce DNA adsorption.

Magnesium ions induced a weaker adsorption on mica than spermidine, a natural trivalent polyamine, because of its lower valence and also its larger radius of gyration *i*.*e*. spermidine was a linear molecule which allowed a closer approach between DNA and mica than globular magnesium. Monovalent salt (NaCl 50 mM) was also added to the buffer to reduce the adsorption strength since monovalent cations then partially replaced multivalent cations on both DNA and mica surfaces.

The use of a low spermidine concentration (10 µM spermidine, 50 mM NaCl, 10 mM Tris-HCl pH 7.5) as incubation buffer led to a significantly lower surface density of hybridized DNA than for magnesium (10 mM MgCl₂, 20mM Tris-HCl pH 7.5, 50 mM NaCl), even for long incubation times. Increasing the spermidine concentration induced a lower efficiency of hybridization and a longer time was required to reach the plateau.

The inventors thus concluded that increasing the adsorption strength was not favorable for surface hybridization on mica. It lowered the kinetics and favored the formation of multiplexes or partial hybridization.

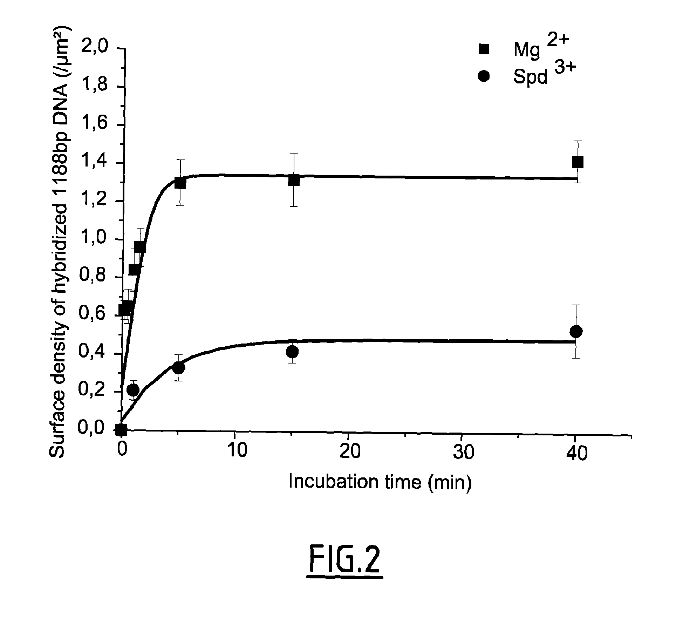

The inventors then studied the influence of the incubation time on the hybridization efficiency in 10 mM MgCl₂, 20 mM Tris-HCl pH 7.5, 50 mM NaCl. The surface density of hybridized fragment increased with the incubation time up to 5 min and then reached a plateau value (**Figure 2**). At incubation times shorter than 5 min, fully hybridized DNA, single ssDNA and partially hybridized ssDNA multiplexes were observed. Above 5 min incubation, isolated ssDNA fragments disappeared. Consequently, they were completely integrated within the hybridized DNA (70% of the adsorbed bodies) or within the partially hybridized DNA multiplexes (30%). It is worth noting that the probability of multiplexes formation could be significantly decreased with a lower surface density of ssDNA fragment at the beginning of the reaction.

### Effect of fragment length on hybridization efficiency and possible discrimination between probes based on their length

To study the influence of the DNA length on the hybridization efficiency, the inventors measured the efficiency of surface hybridization for a long (4361 bp), intermediate (1188 bp) and short fragments (600 bp) in 10 mM MgCl₂, 20 mM Tris-HCl pH 7.5, 50 mM NaCl at 65°C. The results indicated that the longest fragment was less likely to be hybridized on the mica surface even after 40 min incubation (0.1 hybridized 4361 bp DNA /µm²) whereas a high surface density of hybridized smallest DNA could be obtained (nearly 2 hybridized 600 bp DNA /µm²). Concerning the intermediate length (1188 bp), the hybridization process took place with a relatively high efficiency (around 1.4 hybridized DNA/µm²) for practical applications so that it could be considered as the longest fragment that could be reasonably used for this method.

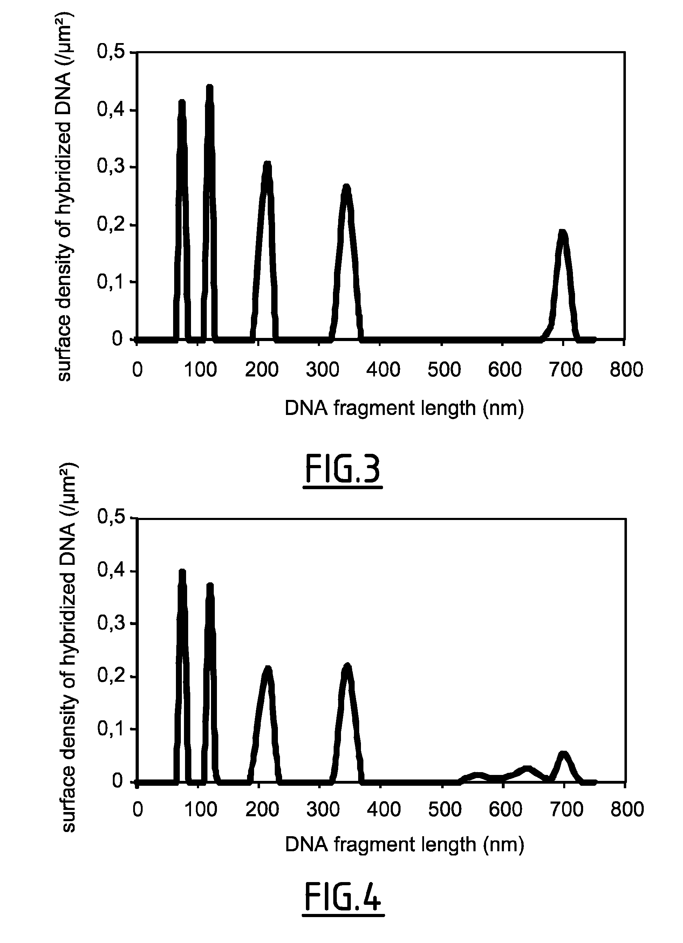

The point of this strategy was that it allowed the use of several probes on a same surface. However it was needed to address the reliability of the detection scheme in presence of many DNA fragments. Five DNA fragments, which lengths ranging from 231 bp to more than 2000 bp, were then denatured, adsorbed on mica, and allowed to hybridize for 5 min in Mg buffer at 65°C. **Figure 3** shows the length distribution of the adsorbed DNA without denaturation process which exhibits five peaks corresponding to each fragment. **Figure 4** shows the length distribution of the adsorbed DNA after denaturation and hybridization process. As long as DNA length was shorter than 400 nm, the width of the peak distribution corresponded to the experimental error *i*.*e*. about 20 nm and thus gave similar results to that obtained when the contour lengths of dsDNA fragments were measured without denaturation process (**Figure 3** compared to **Figure 4**). However, for higher DNA lengths, the peak of the hybridized DNA was broader. For example, the 700 nm fragments induced a larger length distribution, ranging from 500 to 750 nm, which was likely due to incomplete hybridization.

Concerning the lower limit of the DNA length, the hybridization of a 78 nm long DNA was still clearly detectable.

### Conclusion on the results already obtained on DNA hybridization on mica

The inventors thus demonstrated that mica was a unique surface for rapid hybridization of nucleic acids adsorbed on its surface via facilitated diffusion. A very low surface density of probe and target was indeed enough for detection of hybridization at the single molecule level.

Moreover, they provided evidence that measurement of the contour length of hybridized DNA by AFM was a reliable detection scheme, and that it was possible to use several probes on the same spot.

## Claims

1. Use of a negatively charged surface, on which at least one single stranded nucleic acid probe is attached at several points along its length, to specifically hybridize the probe with at least one nucleic acid target.

2. The use according to claim 1, wherein the negatively charged surface is a mineral.

3. The use according to claim 1 or 2, wherein the negatively charged surface is an aluminosilicate.

4. The use according to any of claims 1 to 3, wherein the negatively charged surface is mica.

5. The use according to any of claims 1 to 4, wherein attachment of the probe to the surface is mediated by non-covalently bound cations.

6. The use according to claim 5, wherein the cations are selected from the group consisting of monovalent, divalent, trivalent and tetravalent cations.

7. The use according to claim 5 or 6, wherein the cations are selected from the group consisting of Na⁺, K⁺, Li⁺, Zn²⁺, putrescine, Al³⁺, Fe³⁺, Cobalt hexamine, Mg²⁺, Mn²⁺, Ni²⁺, Ca²⁺, Cd ²⁺ spermidine, and spermine.

8. The use according to any of claims 1 to 7, wherein the single-stranded nucleic acid probe is constituted of DNA.

9. The use according to any of claims 1 to 7, wherein the single-stranded nucleic acid probe is constituted of RNA.

10. The use according to any of claims 1 to 9, wherein the single-stranded nucleic acid probe comprises from 100 to 2000 bases.

11. The use according to any of claims 1 to 10, wherein a plurality of single-stranded nucleic acid probes is attached to the surface.

12. The use according to claim 11, wherein at least two different single-stranded nucleic acid probes are attached to the surface.

13. The use according to any of claims 1 to 12, wherein the nucleic acid target is single-stranded.

14. The use according to any of claims 1 to 13, wherein the nucleic acid target is constituted of DNA.

15. The use according to any of claims 1 to 13, wherein the nucleic acid target is constituted of RNA.

16. A method for detecting a nucleic acid target in a composition, comprising:
- contacting a composition liable to contain the nucleic acid target, with a surface as defined in any of claims 1 to 12,
- washing the surface so as to discard nucleic acids which are not attached on the surface,
- allowing hybridization of the nucleic acid target with a single-stranded nucleic acid probe to proceed,
- determining if hybrization of the nucleic acid target with a single-stranded nucleic acid probe has occurred,
whereby, if hybridization has occurred, the nucleic acid target is detected.

17. The method according to claim 16, wherein the nucleic acid target is single-stranded.

18. The method according to claim 16 or 17, wherein the nucleic acid target is constituted of DNA.

19. The method according to claim 16 or 17, wherein the nucleic acid target is constituted of RNA.

20. The method according to any of claims 16 to 19, wherein determining if hybridization of the nucleic acid target with a single-stranded nucleic acid probe has occurred, is carried by atomic force microscopy (AFM).


SEQUENCE LISTING

<110>  INSERM

<120>  Method for the detection of specific nucleic acid hybridization

<130>  BET 08P0211

<160>  2

<170>  PatentIn version 3.4

<210>  1
<211>  27
<212>  DNA
<213>  Artificial

<220>
<223>  primer

<400>  1
catagcagaa ctttaaaagt actcatc                                          27


<210>  2
<211>  24
<212>  DNA
<213>  artificial

<220>
<223>  primer

<400>  2
agttgcatga taaagaagac agtc                                             24